(19) Europäisches Patentamt

European Patent Office

Office européen des brevets

(11) **EP 1 451 141 B1**

(12) **EUROPÄISCHE PATENTSCHRIFT**

(45) Veröffentlichungstag und Bekanntmachung des Hinweises auf die Patenterteilung:
**22.11.2006 Patentblatt 2006/47**

(21) Anmeldenummer: **02798307.1**

(22) Anmeldetag: **08.11.2002**

(51) Int Cl.:
*C07C 209/00* (2006.01)   *C07C 211/65* (2006.01)

(86) Internationale Anmeldenummer:
**PCT/EP2002/012528**

(87) Internationale Veröffentlichungsnummer:
**WO 2003/042154 (22.05.2003 Gazette 2003/21)**

(54) **VERFAHREN ZUR HERSTELLUNG VON ALKALIMETALL-DIALKYLAMIDEN**

METHOD FOR PRODUCTION OF ALKALI METAL DIALKYLAMIDES

PROCEDE DE PRODUCTION DE DIALKYLAMIDES DES METAUX ALCALINS

(84) Benannte Vertragsstaaten:
**AT BE BG CH CY CZ DE DK EE ES FI FR GB GR IE IT LI LU MC NL PT SE SK TR**

(30) Priorität: **12.11.2001 DE 10155474**

(43) Veröffentlichungstag der Anmeldung:
**01.09.2004 Patentblatt 2004/36**

(73) Patentinhaber: **BASF Aktiengesellschaft**
**67056 Ludwigshafen (DE)**

(72) Erfinder:
• **STEINBRENNER, Ulrich**
**67435 Neustadt (DE)**
• **BÖHLING, Ralf**
**64347 Griesheim (DE)**
• **FUNKE, Frank**
**68161 Mannheim (DE)**
• **HARDER, Wolfgang**
**69469 Weinheim (DE)**

(74) Vertreter: **Hörschler, Wolfram Johannes**
**Isenbruck, Bösl, Hörschler, Wichmann, Huhn**
**Theodor-Heuss-Anlage 12**
**68165 Mannheim (DE)**

(56) Entgegenhaltungen:
WO-A-86/03744     WO-A-93/14061
WO-A-97/02210     GB-A- 2 280 671
US-A- 2 750 417     US-A- 2 799 705

**Beschreibung**

Die vorliegende Erfindung bezieht sich auf ein Verfahren zur Herstellung von Alkalimetall-Dialkylamiden aus einem primären oder sekundären Alkylamin und Alkalimetall unter Zusatz von 1,3-Butadien als Elektronenüberträger. Durch Einhalten einer bestimmten Konzentration an 1,3-Butadien bezüglich des Amins bei der Zugabe des 1,3-Butadiens wird nur eine geringe Menge an Butadien-Additionsprodukten gebildet. Die mit dem erfindungsgemäßen Verfahren hergestellten Amide eignen sich insbesondere als Katalysatoren bei der Herstellung von Trialkylaminen durch Addition von Olefinen an Dialkylamin, insbesondere der Herstellung von Triethylamin aus Ethylen und Diethylamin.

Zur Herstellung von Alkalimetalldialkylamiden sind aus dem Stand der Technik mehrere Verfahren bekannt. In vielen Fällen wird dabei von einer Verbindung des Alkalimetalls ausgegangen, die mit einem Olefin unter Addition von diesem umgesetzt wird. Bekannt sind etwa die Reaktion von Alkalimetallorganylen, beispielsweise einem Alkalimetallalkyl, mit Dialkylamin, und die Umsetzung von Alkalimetallhydrid mit Dimethylamin.

Die direkte Umsetzung eines Alkalimetalls mit einem Amin ohne Zufügung weiterer, reaktionsbeschleunigender oder katalysierender Substanzen ist dabei nur in bestimmten Fällen möglich, beispielsweise bei Einsatz von reaktiven Aminen. Aromatische Amine, etwa Anilin, lassen sich generell gut mit gängigen Alkalimetallen umsetzen. Bei aliphatischen Aminen ist die Reaktion prinzipiell nur bei Einsatz von Monomethylamin erfolgreich. Eine andere Eduktgruppe, die sich zur Herstellung der entsprechenden Amide durch Umsetzung mit einem Alkalimetall eignet, sind Hexaalkyldisilazane, beispielsweise Hexamethyldisilazan.

Die direkte Umsetzung von Dialkylaminen mit Alkalimetall gelingt dagegen bei Zugabe elektronenliefernder Substanzen. Als solche eignen sich vor allem konjugierte Diene, vorzugsweise 1,3-Butadien, Isopren, Naphthalin und Styrol. Diese Durchführungsweise ermöglicht die Synthese von Dialkylamiden in guten Ausbeuten. Beispiele für derartige Herstellungsverfahren von Amiden sind in den US 2,799,705, US 2,750,417, US 4,595,779 und die WO 93/14061 beschrieben. Ein kritischer Punkt ist hier jedoch die Wahl der elektronenliefernden Substanzen.

Naphthalin und Styrol liefern gute Ergebnisse, stehen jedoch entweder nur in zu geringen Mengen zur Verfügung (Naphthalin) bzw. sind ein wertvolles Ausgangsprodukt für chemische Endprodukte (Styrol). Im Fall von Cyclohexadien und Isopren handelt es sich um Verbindungen, die aus der petrochemischen Synthese nur in untergeordneten Mengen erhältlich und daher schwer verfügbar sind bzw. einen zu hohen Preis für den Einsatz in einem großtechnischen Verfahren aufweisen.

1,3-Butadien ist dagegen ein Produkt, das in großen Mengen preiswert zur Verfügung steht. Dem Einsatz von 1,3-Butadien als elektronenliefernde Substanz in der Synthese von Alkalimetallamiden aus Olefinen und Dialkylaminen steht jedoch häufig entgegen, dass 1,3-Butadien leichter als die anderen elektronenliefernden Substanzen an Dialkylamin addiert. Dabei wird das entsprechende Butenyldialkylamin gebildet. Dieses ist häufig nicht oder nur unter einem unverhältnismäßig großen Aufwand von dem gebildeten Amid zu entfernen und liegt bei Einsatz des Amids als Katalysator in der Synthese von Trialkylamin als unerwünschte Verunreinigung im Endprodukt vor. Insbesondere bei der Synthese von Triethylamin aus Diethylamin und Ethylen lässt sich das Nebenprodukt Butenyldiethylamin nur unter erheblichem Aufwand von dem Produkt Triethylamin abtrennen.

Es ist die Aufgabe der vorliegenden Erfindung, ein Verfahren zur Herstellung von Alkalimetall-Dialkylamiden aus Dialkylamin und Alkalimetall zur Verfügung zu stellen, bei dem 1,3-Butadien als elektronenliefernde Substanz eingesetzt werden kann. Das erhaltene Amid soll im Fall des 1,3-Butadiens nur geringe Verunreinigungen an dem Additionsprodukt Butenyldiethylamin aufweisen und als Katalysator in der Synthese von Trialkylaminen, insbesondere Triethylamin, aus dem entsprechenden Dialkylamin und Olefin einsetzbar sein.

Diese Aufgabe wird gelöst durch ein Anspruch 1.

Vorzugsweise liegt die Konzentration an Dialkylamin bei Werten bis 25 Gew.-%, insbesondere bis 15 Gew.-%. Im Bezug auf 1,3-Butadien ist es bevorzugt, wenn dessen Konzentration im Reaktionsgemisch bei Werten bis 3 Gew.-%, insbesondere bis 1,5 Gew.-%, liegt.

Es wurde gefunden, dass im Fall des 1,3-Butadiens nur eine geringe Menge an Butenyldialkylamin gebildet wird, wenn die Reaktion derart durchgeführt wird, dass Dialkylamin und 1,3-Butadien so zugegeben werden, dass in der Reaktionslösung die vorstehend genannten Konzentrationen der beiden Edukte vorliegen.

Das erfindungsgemäße Verfahren wird also so durchgeführt, dass die Zugabe der beiden Edukte in einer Weise erfolgt, die ein Aufpegeln der Konzentration dieser Edukte über die angegebenen Grenzen hinaus verhindert. Der Reaktionsverlauf kann dabei so gewählt werden, dass die Stationärkonzentration eines oder auch beider Edukte praktisch bei 0 % liegt, also das Edukt bei der Zugabe direkt abreagiert und die Stationärkonzentration bei Werten unterhalb der mit üblichen Geräten erzielbaren Nachweisgrenzen liegt.

Im allgemeinen wird technisches Alkalimetall verwendet, das durch bis zu 10 Gew.-% Oxide, Hydroxide, Calcium und die anderen Alkalimetalle verunreinigt ist. Andere Elemente können in Spuren (< 1 Gew.-%) vorhanden sein, diese stören generell aber auch bei höheren Konzentrationen nicht. Es kann natürlich auch vorgereinigtes Alkalimetall verwendet werden, das die erwähnten Verunreinigungen nicht oder nur in Spuren aufweist. Aus Kostengründen wird generell technisches Alkalimetall bevorzugt. Es können alle Alkalimetalle eingesetzt werden, vorzugsweise werden Li, Na oder K eingesetzt, mehr bevorzugt sind Na oder K, insbesondere Na. Gegebenenfalls können auch Mischungen der Alkalimetalle eingesetzt werden.

Vor der Zugabe in die Reaktion wird das Alkalimetall in einem geeigneten inerten Lösungsmittel dispergiert. Als inerte Lösungsmittel werden vorzugsweise gesättigte Kohlenwasserstoffe verwendet, vorzugsweise leichtsiedende Paraffine, wie n-Butan, i-Butan, Pentane und Hexane, Cyclohexan und deren Gemische oder hochsiedende, gegebenenfalls verzweigte und gesättigte Cycloparaffine enthaltende Paraffine, beispielsweise Weißöl. Andere geeignete inerte Lösungsmittel sind Monoolefine, vorzugsweise n-Butene, Isobuten, Pentene und Hexene. Ein anderes geeignetes Lösungsmittel sind Trialkylamine. Da das erfindungsgemäß hergestellte Amid bzw. die in dem erfindungsgemäßen Verfahren erhaltene Lösung dieses Amids in einer bevorzugten Verwendung, die ebenfalls Gegenstand der vorliegenden Erfindung ist, als Katalysator bei der Herstellung von Trialkylamin eingesetzt wird, wird bei Wahl von Trialkylamin als Lösungsmittel vorzugsweise das Trialkylamin verwendet, das in der nachfolgenden Trialkylamin-Synthese erhalten wird.

Die erwähnten Lösungsmittel sind in der Regel technischen Ursprungs und können auch acide Verunreinigungen wie beispielsweise Wasser, Aldehyde, Ketone, Amide, Nitrile oder Alkohole in geringen Mengen enthalten.

Die Dispersion kann oberhalb der Schmelztemperatur des Alkalimetalls mit beispielsweise einem geeigneten Rührer, einer Düse, einer Reaktionsmischpumpe oder einer Pumpe und einem statischen Mischer erfolgen. Das Alkalimetall kann auch in kaltes Lösungsmittel eingedüst oder aus der Gasphase auf kaltes Lösungsmittel gesprüht werden. Auch das Versprühen in kaltes Gas mit anschließender Redispersion ist möglich.

Weitere Möglichkeiten bestehen darin, das Alkalimetall in einer Mischung aus inertem Lösungsmittel und Eduktamin zu dispergieren oder das Alkalimetall in dem oder den Lösungsmittel zu dispergieren und das entsprechende Eduktamin zuzugeben. Gegebenenfalls wird für den Dispergiervorgang eine separate Vorrichtung eingesetzt, beispielsweise ein Rührkessel, eine Düse oder eine Reaktionsmischpumpe.

Bei der Herstellung des Amids wird das Alkalimetall generell in Form von feinen Partikeln vorgelegt. Im Fall von Natrium weisen diese Partikel vorzugsweise eine Größenverteilung derart auf, dass 50 Gew.-% der Partikel in einer Größe von < 1000 $\mu$m, mehr bevorzugt < 300 $\mu$m, insbesondere < 100 $\mu$m vorliegen.

In einer Ausführungsform der vorliegenden Erfindung wird das Alkalimetall in einem Paraffin dispergiert und dieses Paraffin vor dem Einsatz des Alkalimetalls in die Reaktion zumindest zum großen Teil abdekantiert und gegen Trialkylamin und/oder Dialkylamin ersetzt.

Anschließend wird 1,3-Butadien entweder allein oder im Gemisch mit dem Edukt-Dialkylamin zudosiert. Alternativ ist auch eine gleichzeitige Zugabe des 1,3-Butadiens und des Dialkylamins möglich.

Das eingesetzte Dialkylamin und 1,3-Butadien können in technischer Qualität oder gereinigt eingesetzt werden. Dialkylamine können beispielsweise verunreinigt sein durch geringe Mengen an Wasser, Monoalkylamin, Alkoholen, Nitrilen, Amiden, N-Alkylidenalkyliminen und anderen Di- und Trialkylaminen. 1,3-Butadien kann verunreinigt sein durch Wasser, andere $C_4$-Kohlenwasserstoffe, insbesondere Di- und Oligomerisationsprodukte des 1,3-Butadiens wie beispielsweise 1,2-Divinylcyclobutadien, 4-Vinyl-cyclohex-1-en, 1,5-Cyclooctadien und ist üblicherweise durch geringe Mengen an Radikalfängern stabilisiert. Anstelle von 1,3-Butadien können auch 1,3-butadienhaltige Kohlenwasserstoffgemische eingesetzt werden, beispielsweise C4-Schnitte, wie sie beim Cracken von Naphtha, bei der Dehydrierung von LPG oder LNG oder der Fischer-Tropsch-Synthese anfallen. Saure Verunreinigungen wie Wasser, Alkohole, oder C-H-acide Verbindungen wie beispielsweise Nitrile, Amide oder Alkine stören nicht. Es kann unter Umständen ein Ausbeuteverlust beobachtet werden.

Vorzugsweise werden ca. 5 bis 10 % des 1,3-Butadiens bzw. des 1,3-Butadien/Dialkylamin-Gemisches zu Beginn zugegeben und das Anspringen der Reaktion abgewartet. Bei der Herstellung des Amidkatalysators aus elementarem Metall, vorzugsweise Natrium, wird eine Temperatur von -30 bis 90°C, vorzugsweise 0 bis 70°C, insbesondere 30 bis 50°C eingehalten.

Bezüglich der erfindungsgemäßen Konzentrationen von Dialkylamin und Butadien im Reaktionsgemisch ist es nicht nötig, dass die Konzentration über die gesamte Reaktion bei einem stationären Wert liegt. Schwankungen sind innerhalb der vorstehend angegebenen Werte - also auch der bevorzugten und der insbesonderen Werte - praktisch ohne Auswirkungen auf den Reaktionsverlauf.

Ein weiterer Parameter des erfindungsgemäßen Verfahrens der Alkalimetallamidherstellung, der einen Einfluss auf die Produktverteilung haben kann, ist das molare Verhältnis von 1,3-Butadien zu dem eingesetzten Alkalimetall. Dieses beträgt vorzugsweise 0,5 bis 1,2, mehr bevorzugt 0,5 bis 1,0, insbesondere 0,5 bis 0,7.

Die gesamte Synthese kann diskontinuierlich oder kontinuierlich in einem Rührkessel bzw. kontinuierlich in einem Strömungsrohr, einem Schlaufenreaktor oder einer Rührkesselkaskade durchgeführt werden. Bei der kontinuierlichen Synthese wird beispielsweise das Alkalimetall kontinuierlich in einen Strom aus Trialkylamin eingedüst, dieser Strom gekühlt und über Zwischeneinspeisungen 1,3-Butadien und Dialkylamin zugegeben.

Nach der Umsetzung des Alkalimetalls mit dem Dialkylamin unter den vorstehend beschriebenen Bedingungen enthält man eine Suspension des hergestellten Alkalimetalldialkylamids in dem eingesetzten Lösungsmittel bzw. dem Lösungsmittelgemisch. Neben dem Amid und dem oder den Lösungsmitteln enthält das Gemisch unterschiedliche Mengen an Dialkylamin, Butenyldialkylamin (aus der Addition des 1,3-Butadiens) und Butene. Dieses Gemisch kann von den unerwünschten Verunreinigungen befreit und dann als Katalysator in der Hydroaminierungsreaktion eingesetzt werden. Vorteilhafterweise setzt man dieses Gemisch jedoch als solches in der Hydroaminierungsreaktion ein. Durch eine geringe Bildung von Butenyldialkylaminen, bei denen es sich generell um N-But-3-endialkylamin, N-But-(E,Z)-2-endialkylamin und N-But-(E,Z)-1-endialkylamin in unterschiedlichen Mengenverhältnissen handelt, enthält das bei der Hydroaminierung hergestellte Trialkylamin auch nur geringe Mengen der Butenylsubstituierten Amine.

Man erhält ein Gemisch aus Alkalimetalldialkylamid, gegebenenfalls eingesetztem Lösungsmittel und sekundärem Amin/Aminen. Dieses direkt nach der Reaktion erhaltene Gemisch weist dabei ein molares Verhältnis sämtlicher erhaltener Hydroaminierungsprodukte zu dem Alkalimetalldialkylamid, berechnet als Monomer $MNR_2$, mit M = Alkalimetall, von < 1,5, vorzugsweise < 1, insbesondere < 0,3 auf.

Das erfindungsgemäße Verfahren der Amidherstellung bzw. der nachfolgenden Hydroaminierung eignet sich generell für den Einsatz von Aminen (als Ausgangsprodukt für die Diamid- und Trialkylaminherstellung) mit $C_1$-$C_{50}$-Alkylgruppen. Die Alkylgruppen können linear oder verzweigt und acyclisch oder cyclisch sein sowie gegebenenfalls einen oder mehrere inerte Substituenten aufweisen. Ebenfalls möglich ist der Einsatz von Aminen mit Substituenten, die aromatische oder olefinische Substituenten aufweisen, so lange sich diese Substituenten nicht in Konjugation zum Stickstoff befinden. Beispiele für derartige Substituenten sind Benzyl, Phenethyl, Hex-4-enyl und Allyl. Das erfindungsgemäße Verfahren bietet Vorteile insbesondere bei solchen Aminen, die sterisch wenig anspruchsvolle Substituenten aufweisen, da die Tendenz zur Addition des 1,3-Butadiens an das Amid bei geringer werdendem Raumbedarf der Substituenten am Stickstoffatom zunimmt. Vorzugsweise sind die Alkylgruppen ausgewählt aus Methyl, Ethyl, n-Propyl, i-Propyl, n-Butyl, sec-Butyl, i-Butyl, tert-Butyl, n-Pentyl, i-Pentyl, Decyl, Dodecyl, Hexadecyl, Cyclohexyl, Cyclopentyl, wobei unter diesen Alkylresten diejenigen bevorzugt sind, aus denen Alkylamine mit einem Wasserstoffatom in β-Stellung zum Stickstoffatom resultieren. Insbesondere sind die Alkylgruppen ausgewählt aus Ethyl und n-Butyl. In der meistbevorzugten Ausführungsform der vorliegenden Erfindung ist das Eduktamin Diethylamin.

Das erfindungsgemäße Verfahren eignet sich auch zur Herstellung der Alkalimetallamide von Monoalkylaminen. Diese weisen im Vergleich zu Dialkylaminen bei der Umsetzung mit Alkalimetallen häufig unterschiedliche Reaktivitäten auf. Dies ist zum großen Teil auf die unterschiedlichen sterischen Anforderungen der Monoalkylamine gegenüber dem Dialkylamin mit gleichen Alkylgruppen wie in dem entsprechenden monosubstituierten Amin zurückzuführen. Monomethylamin nimmt insofern eine Sonderstellung ein, als dass es auch ohne Zugabe einer elektronenliefernden Substanz mit Alkalimetallen unter Amidbildung reagiert.

Die Hydroaminierung unter Verwendung der erfindungsgemäß hergestellten Amide als Katalysator wird unter den üblichen, dem Fachmann bekannten Bedingungen bezüglich Druck und Temperatur durchgeführt. Die Temperaturen liegen generell bei Werten von 30 bis 180°C, vorzugsweise 50 bis 100°C, und Drücken von 1 bis 200 bar.

Die Olefine, mit denen das Eduktamin umgesetzt wird, sind generell Olefine mit einer C-Zahl von 2 bis 20. Vorzugsweise werden als Olefine Ethylen, Propylen, 1-Buten, 2-Buten oder Cyclohexen eingesetzt, insbesondere ist das Olefin Ethylen.

Durch das erfindungsgemäße Verfahren lässt sich die Alkalimetalldialkylamidherstellung aus Alkalimetall und dem entsprechenden Dialkylamin unter Verwendung des preiswert verfügbaren 1,3-Butadiens durchführen, ohne dass eine unzumutbar große Menge an Butadien-Additionsprodukt gebildet wird. Das erfindungs gemäße Verfahren lässt sich auch unter Verwendung anderer bekannter elektronenliefernder Substanzen, beispielsweise Isopren, Cyclohexadien, Naphthalin oder Styrol durchführen. Da die genannten Substanzen weniger als 1,3-Butadien zur Addition an Aminen neigen, sind die erhaltenen Vorteile gegenüber den bisherigen Verfahren bei Verwendung anderer elektronenliefernder Substanzen in dem erfindungsgemäßen Verfahren im allgemeinen geringer.

Die Erfindung wird nun in den nachfolgenden Beispielen erläutert.

**Beispiele**

**Allgemeines**

Alle verwendeten Lösungsmitten wurden mit 3A-Molsieb über Nacht getrocknet. Alle Arbeiten fanden unter Argon als Schutzgas statt.

Technisches Natrium wurde mit einem Ultraturrax® in n-Dodecan bei 150°C dispergiert, dann wurde ohne Rühren abgekühlt (50 Gew.-% < 280 μm Tröpfchendurchmesser). Anschließend wurde das Na abzentrifugiert, in dem gemischten Lösungsmittel [Di- oder Triethylamin ("DEA oder "TEA") oder n-Heptan] aufgeschlämmt und wieder abzentrifugiert. Dieser Prozess wurde so lange wiederholt, bis < 1 Gew.-% Dodecan im Lösungsmittel vorlagen.

Anschließend wurde das Natrium in der angegebenen Menge des jeweiligen Lösungsmittels aufgeschlämmt, eine definierte Menge an n-Undecan als interner Standard zugegeben und das Gemisch in einen Rührkesselreaktor von 800 ml Inhalt überführt, mit 3 bar Argon beaufschlagt, auf die gewünschte Temperatur thermostatisiert und Diethylamin und 1,3-Butadien wie unten beschrieben flüssig zudosiert.

Während der Reaktion wurden über einem Filter mit 7 μm Porenweite Proben von ca. 3 ml entnommen, mit einem Tropfen 50 % wässriger KOH versetzt und die organische Phase mittels GC analysiert.

Nach Beendigung der Reaktion wurde die NaNEt$_2$-Ausbeute bestimmt.

Der Verlauf der Reaktion kann über die Bildung von Butenen (1- und 2-Butene) verfolgt werden. Näherungsweise gilt die Reaktionsgleichung:

$$2\,Na + 2\,HNEt_2 + C_4H_6 \Rightarrow 2\,NaNEt_2 + 2\,C_4H_8$$

Über den Gehalt an Butenen können somit Umsatz und Selektivität abgeschätzt werden.

In den nachfolgenden Tabellen sind die unter "Auswertung" angegebenen Gew.-% gerundete Werte, die sich aus den nach GC-Analyse erhaltenen Menge in [g] ergeben.

**Vergleichsbeispiel 1**

Gestartet wurde mit einer Dispersion von 0,5 mol Na in 302 g DEA und 12,165 g n-Undecan. Dann wurden bei 30°C eine Mischung aus 0,55 mol 1,3-Butadien und 0,55 mol DEA zudosiert (6 min ca. 5 %, 29 min warten, dann 135 min restliche 95 %). Es ergab sich der folgende Reaktionsverlauf:

Tabelle 1

| Laufzeit [min] | 6 | 35 | 60 | 85 | 115 | 145 | 170 | 205 |
|---|---|---|---|---|---|---|---|---|
| Zugabe DEA [g] | 2,0 | 2,0 | 7,7 | 15,6 | 24,7 | 32,9 | 40,3 | 40,3 |
| Zugabe Butadien [g] | 1,5 | 1,5 | 5,9 | 11,7 | 18,2 | 24,4 | 29,8 | 29,8 |
| GC-Analysen [g] | | | | | | | | |
| Butadien | <0,0001 | <0,0001 | <0,0001 | 0,033 | 0,029 | 0,045 | 0,066 | 0,028 |
| Butene | 0,18 | 0,16 | 0,50 | 0,78 | 1,4 | 1,5 | 1,5 | 1,6 |
| BueDEA | <0,0001 | 0,24 | 2,4 | 20 | 32 | 47 | 71 | 66 |
| Konzentrationen | | | | | | | | |
| Butadien [Gew.-%] | <0,001% | <0,001% | <0,001% | 0,010% | 0,008% | 0,012% | 0,017% | 0,007% |
| DEA [Gew.-%] | 96% | 96% | 95% | 90% | 87% | 83% | 78% | 79% |
| BueDEA = Butenyldiethylamin | | | | | | | | |

Die Ausbeute an NaNEt$_2$ betrug 7 %, das molare Verhältnis von Butenyldiethylamin zu NaNEt$_2$ betrug ca. 15.

**Vergleichsbeispiel 2**

Gestartet wurde mit einer Dispersion von 0,5 mol Na in 300 g DEA und 10,315 g n-Undecan. Dann wurden bei 10°C eine Mischung von 0,55 mol 1,3-Butadien und 0,55 mol DEA zudosiert (15 min ca. 5 %, 25 min warten, dann 150 min. restliche 95 %). Es ergab sich der folgende Reaktionsverlauf:

Tabelle 2

| Laufzeit [min] | 16 | 73 | 108 | 135 | 160 | 190 | 243 |
|---|---|---|---|---|---|---|---|
| Zugabe DEA [g] | 2,0 | 11,1 | 20,5 | 28,1 | 34,9 | 39,6 | 39,6 |
| Zugabe Butadien [g] | 1,5 | 8,0 | 14,8 | 20,2 | 25,2 | 29,8 | 29,8 |
| GC-Analysen [g] | | | | | | | |
| Butadien | 0,40 | <0,0001 | <0,0001 | <0,0001 | <0,0001 | <0,0001 | <0,0001 |
| Butene | <0,0001 | 0,76 | 1,0 | 1,2 | 1,3 | 1,6 | 1,8 |
| BueDEA | <0,0001 | 11 | 22 | 38 | 46 | 61 | 64 |

(fortgesetzt)

| Konzentrationen Butadien [Gew.-%] | 0,13% | <0,001% | <0,001% | <0,001% | <0,001% | <0,001% | <0,001% |
|---|---|---|---|---|---|---|---|
| DEA [Gew.-%] | 97% | 93% | 90% | 86% | 84% | 80% | 80% |
| BueDEA = Butenyldiethylamin | | | | | | | |

Die Ausbeute an $NaNEt_2$ betrug 10 %, das molare Verhältnis von Butenyldiethylamin zu $NaNEt_2$ betrug ca. 10.

**Vergleichsbeispiel 3**

Gestartet wird mit einer Dispersion von 0,5 mol Na in 110g TEA, 112,6 g DEA und 11,40 g n-Undecan. Dann werden bei 30°C eine Mischung aus 0,55 mol Butadien und 1,65 mol DEA kontinuierlich zudosiert (27 min 5 %, 30 min warten, dann 135 min restliche 95 %). Die zeitaufgelösten Proben ergeben.

Tabelle 3

| Laufzeit [min] | 27 | 58 | 95 | 120 | 150 | 180 | 192 | 240 |
|---|---|---|---|---|---|---|---|---|
| Zugabe DEA [g] | 6,0 | 6,0 | 38,4 | 59,1 | 84,8 | 110,1 | 120,7 | 120,7 |
| Zugabe Butadien [g] | 1,5 | 1,5 | 9,4 | 14,7 | 20,8 | 27,0 | 29,7 | 29,7 |
| GC-Analysen [g] | | | | | | | | |
| Butadien | 1,2 | <0,0001 | <0,0001 | 0,026 | 0,021 | <0,0001 | <0,0001 | <0,0001 |
| Butene | <0,0001 | 0,56 | 1,5 | 2,7 | 4,6 | 5,5 | 6,3 | 7,8 |
| BueDEA | <0,0001 | 2,3 | 9,4 | 21 | 31 | 53 | 63 | 54 |
| Konzentrationen Butadien [Gew.-%] | 0,48% | <0,001% | <0,001% | 0,008% | 0,006% | <0,001% | <0,001% | <0,001% |
| DEA [Gew.-%] | 48% | 48% | 49% | 49% | 49% | 49% | 51% | 50% |

Die Ausbeute an $NaNEt_2$ betrug 70 %, das molare Verhältnis von Butenyldiethylamin zu $NaNEt_2$ ca. 1,2.

**Vergleichsbeispiel 4**

Gestartet wurde mit einer Dispersion von 0,5 mol Na in 308 g TEA und 10,737 g n-Undecan. Dann wurden bei 30°C eine Mischung aus 0,95 mol 1,3-Butadien und 0,95 mol DEA zudosiert (9 min ca. 5 %, 36 min warten, dann 255 min ca. 95 %). Es ergab sich der folgende Reaktionsverlauf:

Tabelle 4

| Laufzeit [min] | 9 | 42 | 72 | 109 | 135 | 170 | 212 | 252 | 271 | 300 | 362 |
|---|---|---|---|---|---|---|---|---|---|---|---|
| Zugabe DEA [g] | 3,5 | 3,5 | 14,1 | 29,4 | 39,0 | 44,1 | 56,3 | 64,8 | 66,2 | 73,4 | 73,4 |
| Zugabe Butadien [g] | 2,6 | 2,6 | 10,0 | 20,5 | 28,8 | 27,0 | 34,6 | 43,0 | 44,4 | 51,4 | 51,4 |
| GC-Analysen [g] | | | | | | | | | | | |
| Butadien | 1,3 | 1,7 | 7,6 | 17 | 22 | 23 | 12 | 2,3 | 1,4 | 1,4 | 0,75 |
| Butene | <0,0001 | <0,0001 | <0,0001 | 0,072 | 0,13 | 0,21 | 0,55 | 0,70 | 0,98 | 5,7 | 6,2 |
| DEA | 2,2 | 2,9 | 13 | 30 | 40 | 41 | 33 | 16 | 13 | 3,4 | 1,2 |
| BueDEA | <0,0001 | <0,0001 | <0,0001 | <0,0001 | <0,0001 | 0,15 | 42 | 85 | 95 | 90 | 95 |
| Konzentrationen | | | | | | | | | | | |
| Butadien [Gew.-%] | 0,41% | 0,54% | 2,2% | 4,6% | 5,8% | 5,8% | 3,0% | 0,55% | 0,33% | 0,33% | 0,17% |
| DEA [Gew.-%] | 0,68% | 0,89% | 3,9% | 8,1% | 11% | 11% | 8,1% | 3,8% | 3,1% | 0,82% | 0,30% |
| BueDEA = Butenyldiethylamin | | | | | | | | | | | |

Die Ausbeute an $NaNEt_2$ betrug 50 %, das molare Verhältnis von Butenyldiethylamin zu $NaNEt_2$ betrug ca. 2,7.

**Vergleichsbeispiel 5**

Gestartet wird mit einer Dispersion von 0,5 mol Na in 223,8 g TEA und 11,215 g n-Undecan. Dann wurden bei 30°C eine Mischung aus 0,55 mol Butadien und 1,65 mol DEA kontinuierlich zudosiert (7 min 5 %, 10 min warten, dann 133 min restliche 95 %). Es ergab sich der folgende Reaktionsverlauf:

Tabelle 5

| Laufzeit [min] | 7 | 17 | 40 | 65 | 83 | 115 | 140 | 150 | 165 | 300 |
|---|---|---|---|---|---|---|---|---|---|---|
| Zugabe DEA [g] | 6,2 | 6,2 | 26,0 | 45,9 | 62,1 | 89,5 | 113,2 | 120,6 | 120,6 | 120,6 |
| Zugabe Butadien [g] | 1,5 | 1,5 | 6,0 | 10,1 | 15,0 | 22,1 | 27,2 | 29,8 | 29,8 | 29,8 |
| GC-Analysen [g] | | | | | | | | | | |
| Butadien | 2,0 | 0,4 | 4,6 | 7,0 | 7,7 | 15 | 17 | 22 | 21 | <0,0001 |
| Butene | 0,077 | 0,38 | 2,4 | 3,6 | 6,5 | 6,6 | 7,2 | 7,0 | 6,9 | 13 |
| DEA | 1,0 | 3,2 | 8,3 | 10 | 24 | 22 | 56 | 49 | 86 | 76 |
| BueDEA | <0,0001 | <0,0001 | 0,11 | 0,19 | 0,55 | 0,75 | 5,7 | 4,1 | 4,3 | 44 |
| Konzentrationen | | | | | | | | | | |
| Butadien [Gew.-%] | 0,82% | 0,17% | 1,8% | 2,5% | 2,6% | 4,5% | 4,8% | 6,0% | 5,8% | <0,001% |
| DEA [Gew.-%] | 0,44% | 1,4% | 3,4% | 4,2% | 9,0% | 8,3% | 18% | 16% | 25% | 21% |

BueDEA = Butenyldiethylamin

Die Ausbeute an NaNEt$_2$ betrug 95 %, es ergab sich Na-Vollumsatz. Das molare Verhältnis von Butenyldiethylamin zu NaNEt$_2$ betrug ca. 0,7. Das nach 170 min aufgepegelte Butadien reagiert rasch ab und bildet hohe Mengen an Butenyldiethylamin.

**Beispiel 1**

Gestartet wurde mit einer Dispersion von 0,55 mol Na in 220 g n-Heptan und 7,001 g n-Undecan. Dann wurden bei 30°C eine Mischung aus 0,35 mol 1,3-Butadien und 1,05 mol DEA kontinuierlich zudosiert (11 min 5 %, 30 min warten, dann 140 min restliche 95 %). Nach 180 min stieg der Gehalt an Buten nicht weiter an. Hier ist die Reaktion beendet. Wird die Reaktion nicht abgebrochen, bildet sich eine erfindungsgemäß unerwünscht hohe Menge an Butenyldiethylamin, wie die Auswertung der Reaktionsmischung nach 230 min ergibt. Es ergab sich der folgende Reaktionsverlauf:

Tabelle 6

| Laufzeit [min] | 11 | 41 | 60 | 90 | 120 | 150 | 180 | 230 |
|---|---|---|---|---|---|---|---|---|
| Zugabe DEA [g] | 3,8 | 3,8 | 11,2 | 27,4 | 43,7 | 60,3 | 76,4 | 76,8 |
| Zugabe Butadien [g] | 0,9 | 0,9 | 2,9 | 6,8 | 10,8 | 14,9 | 18,8 | 18,9 |
| GC-Analysen [g] | | | | | | | | |
| Butadien | 0,55 | 0,023 | <0,0001 | <0,0001 | <0,0001 | 0,077 | 0,35 | 0,053 |
| Butene | 0,041 | 0,92 | 2,3 | 5,2 | 7,7 | 11 | 13 | 12 |
| DEA | 2,1 | 1,0 | 3,2 | 7,7 | 12 | 18 | 28 | 34 |
| BueDEA | <0,0001 | <0,0001 | <0,0001 | <0,0001 | <0,0001 | 0,13 | 4,7 | 13 |
| Konzentrationen | | | | | | | | |
| Butadien [Gew.-%] | 0,24% | 0,010% | <0,001% | <0,001% | <0,001% | 0,027% | 0,12% | 0,018% |
| DEA [Gew.-%] | 0,91% | 0,44% | 1,37% | 3,2% | 4,8% | 6,9% | 10% | 12% |

BueDEA = Butenyldiethylamin

Die Ausbeute an NaNEt$_2$ betrug 85 %, Na-Vollumsatz war erreicht. Das molare Verhältnis von Butenyldiethylamin zu NaNEt$_2$ betrug ca. 0,2 (gemessen nach 180 min).

**Beispiel 2**

Gestartet wurde mit einer Dispersion von 0,5 mol Na in 198 g TEA, 22 g DEA und 11,126 g n-Undecan. Dann wurden bei 30°C eine Mischung aus 0,30 mol 1,3-Butadien und 0,60 mol DEA kontinuierlich zudosiert (9 min 5 %, 30 min warten, dann 140 min restliche 95 %). Es ergab sich der folgende Reaktionsverlauf:

Tabelle 7

| Laufzeit [min] | 9 | 40 | 75 | 105 | 135 | 165 | 180 |
|---|---|---|---|---|---|---|---|
| Zugabe DEA [g] | 2,3 | 2,3 | 11,9 | 21,4 | 30,6 | 39,9 | 44,0 |
| Zugabe Butadien [g] | 0,8 | 0,8 | 4,4 | 7,9 | 11,2 | 14,6 | 16,2 |
| GC-Analysen [g] | | | | | | | |
| Butadien | 0,33 | <0,0001 | <0,0001 | 0,077 | 0,056 | 0,17 | 0,20 |
| Butene | 0,72 | 1,1 | 1,9 | 4,9 | 8,9 | 11 | 11 |
| DEA | 21 | 21 | 21 | 21 | 22 | 24 | 25 |
| BueDEA | 0,039 | 0,044 | 0,12 | 0,90 | 0,75 | 2,6 | 5,8 |
| Konzentrationen | | | | | | | |
| Butadien [Gew.-%] | 0,14% | <0,001% | <0,001% | 0,031% | 0,022% | 0,063% | 0,075% |

(fortgesetzt)

| Konzentrationen DEA [Gew.-%] | 10% | 10% | 10% | 10% | 10% | 10% | 11% |
|---|---|---|---|---|---|---|---|

BueDEA = Butenyldiethylamin

Die Ausbeute an NaNEt$_2$ betrug 77 %, Na-Vollumsatz war erreicht. Das molare Verhältnis von Butenyldiethylamin zu NaNEt$_2$ betrug ca. 0,25.

**Beispiel 3**

Gestartet wird mit einer Dispersion von 1 mol Na in 270 g TEA, 30 g DEA und 11,134 g n-Undecan. Dann werden bei 50°C eine Mischung aus 0,6 mol Butadien und 1,2 mol DEA kontinuierlich zudosiert (32 min 5 %, 30 min warten, dann 221 min restliche 95 %). Die zeitaufgelösten Proben ergeben:

Tabelle 8

| Laufzeit [min] | 32 | 62 | 100 | 130 | 160 | 220 | 250 | 283 | 403 |
|---|---|---|---|---|---|---|---|---|---|
| Zugabe DEA [g] | 4,4 | 4,4 | 17,3 | 28,0 | 38,8 | 63,5 | 74,4 | 87,8 | 87,8 |
| Zugabe Butadien [g] | 1,6 | 1,6 | 6,1 | 10,2 | 14,3 | 23,4 | 27,6 | 32,5 | 32,5 |
| GC-Analysen [g] | | | | | | | | | |
| Butadien | 0,26 | <0,0001 | 1,8 | 1,9 | 0,26 | 0,25 | 0,054 | 0,066 | <0,0001 |
| Butene | 0,46 | 1,2 | 2,0 | 3,7 | 8,5 | 16 | 20 | 23 | 26 |
| DEA | 36 | 37 | 45 | 46 | 46 | 48 | 47 | 47 | 50 |
| BueDEA | 0,23 | 0,65 | 1,5 | 4,7 | 6,9 | 10 | 8,7 | 11 | 17 |
| Konzentrationen | | | | | | | | | |
| Butadien [Gew.-%] | 0,082% | <0,001% | 0,54% | 0,55% | 0,075% | 0,068% | 0,014% | 0,016% | <0,001% |
| DEA [Gew.-%] | 11% | 12% | 14% | 14% | 13% | 13 % | 13% | 13% | 13% |

BueDEA = Butenyldiethylamin

Die Ausbeute an NaNEt$_2$ betrug 88 %, Na-Vollumsatz war erreicht. Das molare Verhältnis von Butenyldiethylamin zu NaNEt$_2$ war ca. 0,15.

Tabelle 9

| Zusammenfassung der Versuche | | | | | | | | |
|---|---|---|---|---|---|---|---|---|
| | **VB1** | **VB2** | **VB3** | **VB4** | **VB5** | **B1** | **B2** | **B3** |
| Na [mol] | 0,5 | 0,5 | 0,5 | 0,5 | 0,5 | 0,55 | 0,5 | 1 |
| dispergiert in | DEA | DEA | DEA/ TEA | TEA | TEA | Heptan | DEA/ TEA | DEA/ TEA |
| **Reaktion** | | | | | | | | |
| Temp.[°C] | 30 | 10 | 30 | 30 | 30 | 30 | 30 | 50 |
| Butadien-Zugabe [mol] | 0,55 | 0,55 | 0,55 | 0,95 | 0,55 | 0,35 | 0,3 | 0,6 |
| DEA-Zugabe [mol] | 0,55 | 0,55 | 1,65 | 0,95 | 1,65 | 1,05 | 0,6 | 1,2 |
| DEA-Konz. [Gew.-%] während der Reaktion | 96-78% | 97-80% | 48-51% | 0,3-11% | 0-25% | 0,44-12% | 10-11% | 11-14% |
| 1,3-Butadien-Konz. [Gew.-%] während der Reaktion | ≤ 0,017 | ≤ 0,13 | ≤ 0,48 | ≤ 5,8 | ≤ 6,0 | ≤ 0,24 | ≤ 0,14 | ≤ 0,55 |
| **Ergebnis** | | | | | | | | |
| Ausbeute (Na) | 7% | 10% | 70% | 50% | 95% | 85% | 77% | 88% |
| Vollumsatz (Na) | nein | nein | nein | nein | ja | ja | ja | ja |
| BDEA/NaNEt$_2$ [mol/mol] | 15 | 10 | 1,2 | 2,7 | 0,7 | 0,2 | 0,25 | 0,15 |

**Patentansprüche**

1. Verfahren zur Herstellung von Dialkylamiden der Alkalimetalle durch Umsetzung des entsprechenden Dialkylamins mit dem entsprechenden Alkalimetall in Gegenwart einer elektronenliefernden Substanz aus der Gruppe 1,3-Butadien, Isopren, Naphthalin und/oder Styrol, wobei für den Fall des 1,3-Butadiens geringe Mengen an Butenyldialkylamin gebildet werden, **dadurch gekennzeichnet, dass** das entsprechende Alkalimetall in einem Lösungsmittel suspendiert und vorgelegt wird und anschließend Dialkylamin und elektronenliefernde Substanz so zugegeben werden, dass das Dialkylamin in einer Menge bis 45 Gew.-%, und die elektronenliefernde Substanz in einer Menge bis 5 Gew.-% vorliegt.

2. Verfahren nach Anspruch 1, **dadurch gekennzeichnet, dass** als elektronenliefernde Substanz 1,3-Butadien eingesetzt wird.

3. Verfahren nach Anspruch 1 oder 2, **dadurch gekennzeichnet, dass** das Alkalimetall ausgewählt ist aus Natrium, Kalium und Lithium.

4. Verfahren nach einem der Ansprüche 1 bis 3, **dadurch gekennzeichnet, dass** das molare Verhältnis von 1,3-Butadien zu dem eingesetzten Alkalimetall 0,5 bis 1,2 beträgt.

5. Verfahren nach einem der Ansprüche 1 bis 4, **dadurch gekennzeichnet, dass** Natrium als Alkalimetall eingesetzt wird und dieses eine Größenverteilung derart aufweist, dass 50 Gew.-% der Partikel in einer Größe von < 1000 $\mu$m

vorliegen.

6. Verfahren nach einem der Ansprüche 1 bis 5, **dadurch gekennzeichnet, dass** das Alkalimetall vor der Reaktion in einem gesättigten Kohlenwasserstoff, Monoolefinen, und/oder einem Trialkylamin suspendiert wird.

7. Verfahren nach einem der Ansprüche 1 bis 6, **dadurch gekennzeichnet, dass** die Alkylgruppen an dem Alkylamin eine Kohlenstoffzahl von $C_1$ bis $C_{50}$ aufweisen, wobei diese Alkylgruppen linear oder verzweigt, acyclisch oder cyclisch sein sowie gegebenenfalls einen oder mehrere inerte Substituenten aufweisen können.

8. Verfahren nach einem der Ansprüche 1 bis 7, **dadurch gekennzeichnet, dass** bei der Herstellung des Amidkatalysators aus elementarem Metall eine Temperatur von -30 bis 90°C eingehalten wird.

9. Gemisch enthaltend Alkalimetalldialkylamid, gegebenenfalls eingesetztes Lösungsmittel und sekundäres Amin/Amine aus dem Verfahren nach einem der Ansprüche 1 bis 8, **dadurch gekennzeichnet, dass** ein molares Verhältnis sämtlicher erhaltener Hydroaminierungsprodukte zu dem Alkalimetalldialkylamid von < 1,5 vorliegt.

10. Verfahren zur Herstellung von Trialkylaminen aus dem entsprechenden Dialkylamin und Olefin, **dadurch gekennzeichnet, dass** als Katalysator ein Gemisch nach Anspruch 9 eingesetzt wird.

11. Verfahren nach Anspruch 10, **dadurch gekennzeichnet, dass** das Olefin, mit dem das Eduktamin umgesetzt wird, ein Olefine mit einer C-Zahl von 2 bis 20 ist.

**Claims**

1. A process for preparing dialkylamides of the alkali metals by reacting the corresponding dialkylamine with the corresponding alkali metal in the presence of an electron-donating substance selected from the group consisting of 1,3-butadiene, isoprene, naphthalene and styrene, with small amounts of butenyldialkylamine being formed in the case of 1,3-butadiene, which comprises suspending the corresponding alkali metal in a solvent and subsequently adding dialkylamine and electron-donating substance in such a way that the dialkylamine is present in an amount of up to 45% by weight and the electron-donating substance is present in an amount of up to 5% by weight.

2. The process according to claim 1, wherein the electron-donating substance used is 1,3-butadiene.

3. The process according to claim 1 or 2, wherein the alkali metal is selected from among sodium, potassium and lithium.

4. The process according to any of claims 1 to 3, wherein the molar ratio of 1,3-butadiene to the alkali metal used is from 0.5 to 1.2.

5. The process according to any of claims 1 to 4, wherein sodium is used as alkali metal and has a size distribution such that 50% by weight of the particles have a size of < 1000 $\mu$m.

6. The process according to any of claims 1 to 5, wherein the alkali metal is suspended in a saturated hydrocarbon, monoolefins and/or a trialkylamine prior to the reaction.

7. The process according to any of claims 1 to 6, wherein the alkyl groups on the alkylamine have from 1 to 50 carbon atoms and may be linear or branched, acyclic or cyclic and may bear one or more inert substituents.

8. The process according to any of claims 1 to 7, wherein the preparation of the amide catalyst from elemental metal is carried out at from -30 to 90°C.

9. The mixture comprising alkali metal dialkylamide, any solvent used and secondary amine/amines from a process according to any of claims 1 to 8, wherein the molar ratio of all hydroamination products obtained to the alkali metal dialkylamide is < 1.5.

10. The process for preparing trialkylamines from the corresponding dialkylamine and olefin, wherein a mixture according to claim 9 is used as catalyst.

**11.** The process according to claim 10, wherein the olefin with which the starting amine is reacted is an olefin having 2 to 20 carbon atoms.

**Revendications**

**1.** Procédé pour la préparation de dialkylamides des métaux alcalins par réaction de la dialkylamine correspondante avec le métal alcalin correspondant en présence d'une substance donneuse d'électrons issue du groupe du 1,3-butadiène, de l'isoprène, de la naphtaline et/ou du styrène, dans lequel, dans le cas du 1,3-butadiène, de faibles quantités de butényldialkylamine sont formées, **caractérisé en ce que** le métal alcalin correspondant est mis en suspension dans un solvant et réservé, et de la dialkylamide et une substance donneuse d'électrons sont ensuite ajoutées de sorte que la dialkylamine soit présente dans une quantité allant jusqu'à 45 % en poids et la substance donneuse d'électrons dans une quantité allant jusqu'à 5 % en poids.

**2.** Procédé selon la revendication 1, **caractérisé en ce que** du 1,3-butadiène est utilisé en tant que substance donneuse d'électrons.

**3.** Procédé selon la revendication 1 ou 2, **caractérisé en ce que** le métal alcalin est choisi parmi le sodium, le potassium et le lithium.

**4.** Procédé selon l'une quelconque des revendications 1 à 3, **caractérisé en ce que** le rapport molaire du 1,3-butadiène sur le métal alcalin utilisé est de 0,5 à 1,2.

**5.** Procédé selon l'une quelconque des revendications 1 à 4, **caractérisé en ce que** le sodium est utilisé en tant que métal alcalin et qu'il présente une répartition dimensionnelle telle que 50 % en poids des particules sont présentes dans une taille < à 1000 $\mu$m.

**6.** Procédé selon l'une quelconque des revendications 1 à 5, **caractérisé en ce que** le métal alcalin est mis en suspension avant la réaction dans un hydrocarbure saturé, des monooléfines, et/ou une trialkylamine.

**7.** Procédé selon l'une quelconque des revendications 1 à 6, **caractérisé en ce que** les groupes alkyle présentent sur l'alkylamine une valeur de carbone de $C_1$ à $C_{50}$, ces groupes alkyle étant linéaires ou ramifiés, acycliques ou cycliques, ainsi que pouvant présenter si nécessaire un ou plusieurs substituants inertes.

**8.** Procédé selon l'une quelconque des revendications 1 à 7, **caractérisé en ce que** l'on respecte une température de -30 à 90°C lors de la préparation du catalyseur amide à partir de métal élémentaire.

**9.** Mélange contenant du dialkylamide de métal alcalin, le cas échéant le solvant utilisé et de l'amine/des amines secondaire(s) issue(s) du procédé selon l'une quelconque des revendications 1 à 8, **caractérisé en ce qu'**il existe un rapport molaire < 1,5 de l'ensemble des produits d'hydroamination obtenus sur le dialkylamide de métal alcalin.

**10.** Procédé pour la préparation de trialkylamines à partir de la dialkylamine correspondante et d'oléfine, **caractérisé en ce que** l'on utilise un mélange selon la revendication 9 en tant que catalyseur.

**11.** Procédé selon la revendication 10, **caractérisé en ce que** l'oléfine, avec laquelle l'amine-éduit réagit, est une oléfine avec une valeur de carbone de 2 à 20.